# EUROPEAN PATENT SPECIFICATION

(11) **EP 3 438 121 B1**
(45) Date of publication and mention of the grant of the patent: **30.09.2020**
(21) Application number: 17900782.8
(22) Date of filing: 31.08.2017
(51) Int. Cl.: C07K 7/16, C07K 1/20, C07K 1/06, C07K 1/02, C07K 5/062

(54) **LIQUID PHASE SYNTHESIS METHOD FOR OXYTOCIN USING POLYPEPTIDES**
FLÜSSIGPHASENSYNTHESEVERFAHREN FÜR OXYTOCIN MITHILFE VON POLYPEPTIDEN
PROCÉDÉ DE SYNTHÈSE EN PHASE LIQUIDE D'OXYTOCINE À L'AIDE DE POLYPEPTIDES

(30) Priority: 17.03.2017 CN 201710158573
(43) Date of publication of application: 06.02.2019
(73) Proprietor: Lanzhou Kaibo Pharmaceutical Co., Ltd., Lanzhou, Gansu 730300 (CN)
(72) Inventor: SUN, Pengcheng, Lanzhou Gansu 730300 (CN); HU, Bihuang, Lanzhou Gansu 730300 (CN)
(74) Representative: Huang, Liwei
(86) International application number: PCT/CN2017/099882
(87) International publication number: WO 2018/166146

(56) References cited:
- WO-A1-2016/154580
- WO-A2-2006/119388
- WO-A2-2011/035330
- CA-A- 798 320
- CN-A- 101 235 081
- CN-A- 101 412 752
- CN-A- 101 914 136
- CN-A- 102 477 082
- CN-A- 105 348 367
- CN-A- 106 967 155
- PETER MARBACH ET AL: "Synthesis of [2-p-Fluorophenylalanine]oxytocin and its Desamino Analogue Using the S-Acetamidomethyl Protecting Group", HELVETICA CHIMICA ACTA, vol. 57, no. 2, 1 February 1974 (1974-02-01), pages 403-414, XP055531916, CH ISSN: 0018-019X, DOI: 10.1002/hlca.19740570215
- J. W. VAN NISPEN ET AL: "Synthesis of fragments of arginine vasopressin and oxytocin containing a cystine residue in position 6", RECUEIL DES TRAVAUX CHIMIQUES DES PAYS-BAS, vol. 103, no. 2, 2 September 1984 (1984-09-02), pages 68-74, XP055531978, Amsterdam, NL ISSN: 0165-0513, DOI: 10.1002/recl.19841030205
- BRUCKDORFER T ET AL: "FROM PRODUCTION OF PEPTIDES IN MILLIGRAM AMOUNTS FOR RESEARCH TO MULTI-TONS QUANTITIES FOR DRUGS OF THE FUTURE", CURRENT PHARMACEUTICAL BIOTECHNO, BENTHAM SCIENCE PUBLISHERS, NL, vol. 5, no. 1, 1 February 2004 (2004-02-01), pages 29-43, XP009063837, ISSN: 1389-2010, DOI: 10.2174/1389201043489620
- SIEBER P ET AL: "Protection of carboxamide functions by the trityl residue. Application to peptide synthesis", TETRAHEDRON LETTERS, ELSEVIER, AMSTERDAM, NL, vol. 32, no. 6, 4 February 1991 (1991-02-04), pages 739-742, XP026648797, ISSN: 0040-4039, DOI: 10.1016/S0040-4039(00)74872-6 [retrieved on 1991-02-04]

## Description

### TECHNICAL FIELD

The present disclosure relates to preparation of polypeptide drugs, in particular to a method for the liquid-phase synthesis of oxytocin, which belongs to the technical field of liquid-phase polypeptide synthesis.

### BACKGROUND

Oxytocin, with a chemical formula of C₄₃H₆₆N₁₂O₁₂S₂ and a molecular weight of 1007.2, is represented by the following chemical structure:

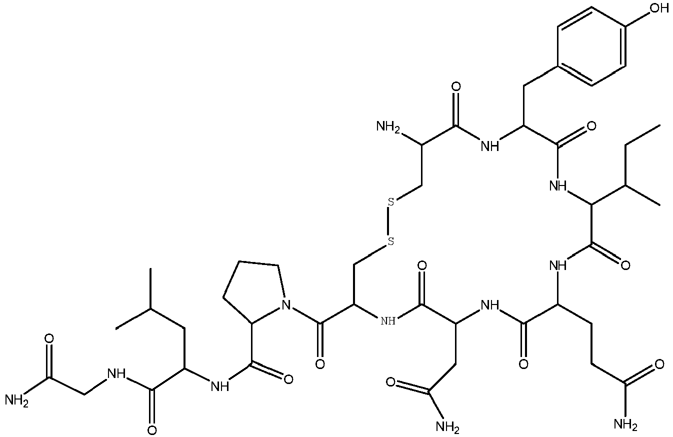

Oxytocin is mainly distributed in the posterior lobe of the hypothalamus of human and mammals, which is clinically used for hasten parturition, postpartum hemorrhage and induction of labor. With the improvement of deep exploration of oxytocin, in addition to its common role in uterine contraction, lactation, etc., it is found that there exist a lot of complicated physiological functions of oxytocin, such as participating in learning and memory process, making influence on drug addiction, social adaptive behavior, maternal behavior, sexual behavior, food intake, pain modulation and regulation of cardiovascular and body temperature, etc.

At present, the domestic bulk drugs of oxytocin are mainly obtainedfrom the extraction of the hypothalamus of pig or bovine, with a biological value of 160 IU/ml. However, there exist biosafety risks in these animal-derived products, besides, naturally occurring impurities, such as vasopressin and the like are difficult to be removed by purification.

So far, methods of liquid-phase synthesis of oxytocin relate to reactions under conditions uncontrollable in safety (such as ammonia-sodium-method), while the reaction steps are numerous and the productivity is low; On the other hand, although introducing solid-phase synthesis simplifies the oxytocin synthetic technology, however, according to literature reported at home and abroad, methods of solid-phase synthesis can still hardly meet the requirements in productivity, purity and potency, among which, the Boc solid-phase synthesis method is gradually discarded in the industry for using hazardous compounds such as hydrogen fluoride and the Fmoc solid-phase synthesis method involves using piperidine as the decapping agent, which is a kind of liquid difficult to be transported and stored and especially as a controlled poison-making chemical, bringing inconveniences to the enterprises in purchase, utilization and material management.

The formation of disulfide bonds, the productivity and purity thereof, which are failing to satisfy the requirements of industrial production becomes another great challenge in the synthesis of oxytocin.

In 1953, oxytocin was initially synthesized by Vincent du Vigneaud using peptide-phase liquid synthesis method which was soon after improved , with its biological activity confirmed. Both of these two methods ultimately employ metal sodium/liquid ammonia to deprotect the protecting group of oxytocin followed by oxidation with air to form disulfide bonds. Other methods to remove protecting groups of the cysteine residues and form disulfide bonds by cyclization include: 1) After completing the synthesis of protected oxytocin linear chain sequence , Photaki employs sodium methylate/methanol to remove the thiol protecting group Bz or Z and 1,2-diiodoethane to form disulfide bonds. Finally, other protective groups were removed with HBr/AcOH to obtain crude oxytocin, which is then purified with a biological value of 380 IU/mg. Mühlemann also adopts 1, 2-diiodoethane as an oxidizing agent to form a disulfide bond with a cyclization yield of 30%; 2) Fujii et al. choose thiol-protected cysteines protected by MBzl and Acm respectively, to synthesize a protected oxytocin linear chain sequence, and futher synthesize to form a disulfide bond to give a crude oxytocin product with yields of 45% and 39%, after removing MBzl and Acm with thallium(III) trifluoroacetate; 3) Akaji et al. choose thiol-protected cysteines protected by Acm, Tacm and But respectively, to synthesize a protected oxytocin linear chain sequence, and futher synthesize to form a disulfide bond with cyclization yields of 56%, 69 % and 64%, after removing Acm, Tacm and But with methyltrichlorosilane and diphenyl sulfoxide respectively. Fernando Albericio conducts a comparative study of oxytocin solid-phase synthesis using the Boc/MBHA resin method and Fmoc/PAL resin method, and further reseach on methods for the formation of disulfide bonds, including air oxidation, iodine oxidation, and oxidation with thallium(III) trifluoroacetate, consequently finding out that thallium(III) trifluoroacetate provides the best yield of 72%, and the yield of iodine oxidation is 28%.

At present, there are mainly liquid-phase method and Fmoc solid-phase method for oxytocin synthesis in the domestic reports. Among which, Chinese invention patent ZL2010102549195 discloses a method for the liquid-phase synthesis of oxytocin, employing metal sodium/liquid ammonia for deprotection followed by oxidation with air to form disulfide bonds to obtain 25% yield, without mention of biological value; Chinese invention patent ZL2008100849408 discloses a liquid phase and solid phase combined method for synthesizing oxytocin, which synthesizes two thiol-protected cysteine residues fragments, performs condensation of these two fragments in 10 to 40 hours and then employs air oxidation method to form a disulfide bond after removing the thiol protecting group Fmoc with piperidine, giving 7-10% yield and 600 IU/ml of biological value, without mention of purity. However, the actual biological value of oxytocin obtained in this method is impossible to be evaluated while using volume calculation, because the amount of oxytocin per milliliter is unknown; Chinese invention patent ZL2005101123565 discloses a method for solid-phase synthesis of oxytocin, using Rink Amide resin as a solid phase carrier , Fmoc-protected amino acid as a monomer, wherein Fmoc-Cys (Trt) -OH as the Fmoc-protected cysteine, and piperidine as a decapping reagent to sequentially synthesize a protected oxytocin peptide chain. The desired peptide chain is then cut off from the resin, and precipitates from the solution to obtain reductive oxytocin when diethyl ether is added. After that, the crude product is oxidized with air or hydrogen peroxide to form a disulfide bond in slight alkaline solution (pH 7-8), giving 21% yield. No disclosure about biological value of oxytocin product is found in this method; Chinese invention patent ZL201210256922X discloses a method for solid-phase synthesis of oxytocin, using Rink Amide resin as a solid phase carrier , Fmoc-protected amino acid as a monomer, Boc-Cys (Trt) -OH as the N-terminal cysteine and piperidine as a decapping reagent to sequentially synthesize a protected oxytocin peptide chain. The desired peptide chain is then cut off from the resin, and precipitates from the solution to obtain uncyclized oxytocin when diethyl ether is added. After that, the crude product is oxidized with air or hydrogen peroxide to form a disulfide bond in slight alkaline solution, giving 33% yield. Also no disclosure about biological value of oxytocin product is found in this method. There are common features of the above-mentioned solid-phase synthesis methods: 1) The cyclization is performed in solution, as well as the formation of disulfide bonds is completed using air oxidation or hydrogen peroxide oxidation, with a yield of up to 33%; 2) N-terminal amino acid undergoes a cyclization reaction without protection (free); 3) Piperidine is employed as a decapping reagent; 4) No biologic value or reference biologic value of oxytocin has been reported.

Besides, "Synthesis of [2-p-Fluorophenylalanine]oxytocin and its Desamino Analogue Using the S-Acetamidomethyl Protecting Group" (PETER MARBACH ET AL, HELVETICA CHIMICA ACTA, vol. 57, no. 2, 1 February 1974, pages 403-414) discloses the synthesis of some analogs of oxytocin, wherein the linear peptides were built up using both stepwise and fragment-condensation procedures, and the S-protecting groups were removed by iodine with simultaneous formation of the disulfide bridge. "FROM PRODUCTION OF PEPTIDES IN MILLIGRAM AMOUNTS FOR RESEARCH TO MULTI-TONS QUANTITIES FOR DRUGS OF THE FUTURE" (BRUCKDORFER T ET AL, CURRENT PHARMACEUTICAL BIOTECHNOLOGY, vol. 5, no. 1, 1 February 2004, pages 29-43) summarized two basic synthesis techniques in peptide synthesis, i.e. Solution Phase Synthesis (SPS) and Solid Phase Peptide Synthesis (SPPS), wherein Fmoc protecting groups are used in the solid phase, i.e. SPPS. WO2006/119388A2 discloses a method of preparing a peptide which is a C-terminal amide derivative and to products thereof, wherein Fmoc technology is also used in SPPS. WO2011/035330A2 also mentions using Fmoc technology in solid phase. "Protection of carboxamide functions by the trityl residue. Application to peptide synthesis" (SIEBER P ET AL, TETRAHEDRON LETTERS, vol. 32, no. 6, 4 February 1991, pages 739-742) mentions using trityl as a protection group and cleavage of trityl by highly concentrated TFA.

In summary, there are still numerous challenges in the synthesis of oxytocin at home and abroad, such as unavoidable use of poison-making chemical like piperidine and ethyl ether, and highly toxic and expensive reagents like thallium(III) trifluoroacetate, uncontrolled conditions (such as sodium metal/liquid ammonia, HF, etc.) and low level of productivity and biological value of oxytocin product.

### SUMMARY

The present invention provides a method for manufacturing oxytocin as described in claim 1 as well as some advantageous embodiments as described in the dependent claims.
In the embodiments of the present invention, a Boc-polypeptide synthesis and Fmoc -polypeptide synthesis combined method is provided, wherein, all the reactions are carried out under mild conditions without using the ammonia-sodium-method decapping reaction which is reported by literature referring to oxytocin liquid-phase synthesis at home and abroad. Further more, the liquid-phase synthesis method of oxytocin is first performed without highly toxic reagents and unsafe reaction conditions, thus greatly reducing the cost of oxytocin synthesis and providing reference for industrial production of oxytocin. Besides, high purity (above 99%) and high biological value (588 IU/mg) of oxytocin is also provided in the embodiments of the present invention.

### BRIEF DESCRIPTION OF THE DRAWINGS

In order to clearly illustrate the technical solution of the embodiments of the disclosure, the drawings of the embodiments will be briefly described in the following, it is obvious that the described drawings are only related to some embodiments of the disclosure and thus are not limitative of the disclosure.
Fig. 1 is a RP-HPLC image of fragment 1: Boc-Cys(Acm)-Tyr(tBu)-OH. Analytical conditions: A: 3% ACN/H₂O, 0.1% TFA, B: ACN, 30-50% B, 0-30min, column: C18, 250x4.6mm, flow rate: 1ml/min, detection wavelength: 210nm;
Fig. 2 is an ESI-MS spectrum of H-Cys(Acm)-Tyr-OH, a strong-acid hydrolysis sample of fragment 1 .
Fig. 3 is a RP-HPLC image of Fmoc-Cys(Acm)-Pro-OMe. Analytical conditions: A: 3% ACN/H₂O, 0.1% TFA, B: ACN, 30-90% B, 0-30 min, column : C18, 250x4.6mm, flow rate: 1ml/min, detection wavelength: 210nm;
Fig. 4 is a RP-HPLC image of H-Cys(Acm)-Pro-OMe. Analytical conditions: A: 3% ACN/H₂O, 0.1% TFA, B: ACN, 0-10% B, 0-30 min, column : C18, 250x4.6mm, flow rate 1ml/min, detection wavelength 210nm;
Fig. 5 is a RP-HPLC image of Fmoc-Asn(Trt)-Cys(Acm)-Pro-OMe. Analytical conditions: A: 3% ACN/H₂O, 0.1% TFA, B: ACN, 50-90% B, 0 -30min, column: C18, 250x4.6mm, flow rate: 1ml/min, detection wavelength: 210nm;
Fig. 6 is a RP-HPLC image of H-Asn(Trt)-Cys(Acm)-Pro-OMe. Analytical conditions: A: 3% ACN/H₂O, 0.1% TFA, B: ACN, 30-90% B, 0 -30min, column: C18, 250x4.6mm, flow rate: 1ml/min, detection wavelength:210nm;
Fig. 7 is a RP-HPLC image of H-Gln(Trt)-Asn(Trt)-Cys(Acm)-Pro -OMe. Analytical conditions: A: 3% ACN/H₂O, 0.1% TFA, B: ACN, 50- 90%B, 0-30min, column: C18, 250x4.6mm, flow rate: 1ml/min, detection wavelength: 210nm;
Fig. 8 is a RP-HPLC image of H-Ile-Gln(Trt)-Asn(Trt)-Cys(Acm)-Pro -OMe (fragment 2). Analytical conditions: A: 3% ACN/H₂O, 0.1% TFA, B: ACN, 50-90% B, 0-30 min, column: C18, 250x4.6 mm, flow rate: 1 ml/min, detection wavelength :210 nm;
Fig. 9 is an ESI-MS spectrum of H-Ile-Gln(Trt)-Asn(Trt)-Cys(Acm)-Pro-OMe (Fragment 2);
Fig. 10 is a RP-HPLC image of Fmoc-Leu-Gly-NH₂. Analytical conditions: A: 3% ACN/H₂O, 0.1% TFA, B: ACN, 60-65% B, 0-30 min, column: C18, 250x4.6mm, flow rate : 1ml/min, detection wavelength: 210nm;
Fig. 11 is a RP-HPLC image of H-Leu-Gly-NH2 (fragment 3). Analytical conditions: A: 3% ACN/H₂O, 0.1% TFA, B: ACN, 0-30% B, 0-30 min, column: C18, 250x4.6mm, flow rate: 1ml/min, detection wavelength: 210nm;
Fig. 12 is an ESI-MS spectrum of H-Leu-Gly-NH₂ (fragment 3);
Fig. 13 is a RP-HPLC image of compound 4. Analytical conditions: A: 3% ACN/H₂O, 0.1% TFA, B: ACN, 75-80% B, 0-30 min, column: C18, 250 x 4.6 mm, flow rate: 1ml/min, detection wavelength: 210nm;
Fig. 14 is a RP-HPLC image of compound 5. Analytical conditions: A: 3% ACN/H₂O, 0.1% TFA, B: ACN, 80-90% B, 0-30 min, column: C18, 250 x 4.6 mm, flow rate: 1ml/min, detection wavelength: 210nm;
Fig. 15 is a RP-HPLC image of compound 6. Analytical conditions: 3% ACN/H₂O, 0.1% TFA, B: ACN, 80-85% B, 0-30 min, column: C18, 250 x 4.6 mm, flow rate: 1ml/min, detection wavelength: 210nm;
Fig. 16 is a RP-HPLC image of compound 7. Analytical conditions: A: 3% ACN/H₂O, 0.1% TFA, B: ACN, 80-85% B, 0-30 min, column: C18, 250 x 4.6 mm, flow rate: 1ml/min, detection wavelength 210nm;
Fig. 17 is a RP-HPLC image of crude oxytocin. Analytical conditions: A: 0.1 M NaH₂PO₄/H₂O, B: 50% CAN/H₂O, 30-60% B, 0-30 min, column: C18, 250 x 4.6 mm , flow rate :1ml/min, detection wavelength: 220nm;
Fig. 18 is a RP-HPLC image of pure oxytocin. Analytical conditions: A: 0.1M NaH₂PO₄/H₂O, B: 50%CAN/H₂O, 30-60%B, 0-30min, column: C18, 250x4. 6mm, flow rate: 1ml/min, detection wavelength: 220nm;
Fig. 19 is an ESI-MS spectrum of pure oxytocin;
Fig. 20 is a HPLC image of Boc-Leu-Gly-NH₂, the intermediate to synthesize fragment 3 according to solution 1. Analytical conditions: A: 3% ACN/H₂O, 0.1% TFA, B: ACN, 10-50% B, 0-30 min, column: C18, 250x4.6mm, flow rate: 1ml/min, detection wavelength: 210nm;
Fig. 21 is a HPLC image of H-Leu-Gly-NH2, fragment 3 synthesized according to solution 1. Analytical conditions: A: 3% ACN/H₂O, 0.1% TFA, B: ACN, 0-30% B, 0-30 min, column: C18, 250x4.6mm, flow rate: 1ml/min, detection wavelength: 210nm.

### DETAILED DESCRIPTION

In order to make objects, technical details and advantages of the embodiments of the disclosure apparent, the technical solutions of the embodiment will be described in a clearly and fully understandable way in connection with the drawings related to the embodiments of the disclosure. It is obvious that the described embodiments are just a part but not all of the embodiments of the disclosure. Based on the described embodiments herein, those skilled in the art can obtain other embodiment(s), without any inventive work, which should be within the scope of the disclosure.

In order to solve the problems existing in the present oxytocin synthesis technology, the invention provides a liquid-phase synthesis technique of oxytocin charactized by high productivity, low cost, safe environment, mild reaction conditions, high level of purity and biological value and great possibility for industrial production. The pure product of oxytocin provided by the invention has a content of 98.1% and a biological value of 588IU/mg measured by the content determination method according to the European Pharmacopoeia (version 8.0).

The method of the embodiments of the present invention comprises the following proceedings:
Fragments 1, 2 and 3 are synthesized in sequence, and then fragments 1 and 2 are assembled to synthesize compound 4, followed by saponification of compound 4 to give compound 5. In the next step, compound 5 and fragment 3 are assembled to obtain a protected oxytocin amino acid sequence (compound 6), which undergoes cyclization , decapping and crystallization in ethyl acetate to give crude oxytocin. At last, the desired product is obtained by C18 RP-HPLC purification.

In one embodiment of the invention, the method for the liquid-phase synthesis of oxytocin comprises the following proceedings:

### 1. Synthesis of fragment 1: Boc-Cys(Acm)-Tyr(tBu)-OH

Boc-Cys(Acm)-OH (1 eq.) and HOSu (1.1 eq.) are dissolved in THF, and then cooled to -10°C in an ice bath for 10 min. To the cool solution is added DCC (1.1 eq.) dissolved in a small amount of THF. After reaction for 20 minutes, H-Tyr(tBu)-OH (1.1 eq.) and NaHCO₃ (1.1 eq.) are dissolve in water and then added to the above solution. The reaction is kept at 15 -25°C, monitored by HPLC, until it is completed. The mixture solution is regulated to neutral by addition of 0.5M HCl, filtrated with removal of insoluble matter, concentrated to remove THF and subsequently regulated to pH=2 by addition of 0.5M HCl. The obtained white solid is then collected by filtration, washed with water until neutral, and then dried in vacuum to give fragment 1.

### 2. Synthesis of fragment 2: H-Ile-Gln(Trt)-Asn(Trt)-Cys(Acm)-Pro-OMe

### (1)Synthesis of compound 2.1: Fmoc-Cys(Acm)-Pro-OMe:

Fmoc-Cys(Acm)-OH (1 eq.) and HOBt (1.1 eq.) are dissolved in the mixture solvent of THF and a small amount of DMF, and then cooled to -10°C in an ice bath for 10 min. To the cool solution is added DCC (1.1 eq.) dissolved in a small amount of THF. After reaction for 20 minutes, H-Pro-OMe.HCl (1.1 eq.), dissolved in THF and well-mixed with TEA (1.1 eq.), is added to the above reaction solution. The reaction is kept at 15 -25°C, monitored by HPLC, until it is completed. The mixture solution is filtrated with removal of insoluble matter, concentrated and subsequently separated solid out by addition of 0.1M HCl. The solid is then collected by filtration, washed to neutral by water, and then dried in vacuum to give compound 2.1.

### (2) Synthesis of compound 2.2: H-Cys(Acm)-Pro-OMe:

The dried compound 2.1 is dissolved in an appropriate amount of diethylamine, concentrated to a minimum amount, precipitated by addition of petroleum ether, filtered, washed with petroleum ether, and dried in vacuum to give compound 2.2.

### (3) Synthesis of Compound 2.3: Fmoc-Asn(Trt)-Cys(Acm)-Pro-OMe:

Fmoc-Asn(Trt)-OH (1 eq.) and HOBt (1.1 eq.) are dissolved in the mixture solvent of THF and a small amount of DMF, and then cooled to -10°C in an ice bath for 10 min. To the cool solution is added DCC (1.1 eq.) dissolved in a small amount of THF. After reaction for 20 minutes, compound 2.2 (1eq.), dissolved in THF, is added to the above reaction solution. The reaction is kept at 15 -25°C, monitored by HPLC, until it is completed. The mixture solution is filtrated with removal of insoluble matter, concentrated and subsequently separated solid out by addition of 0.1M HCl. The solid is then collected by filtration, washed with water until neutral, and then dried in vacuum to give compound 2.3.

### (4) Synthetic Compound 2.4: H-Asn(Trt)-Cys(Acm)-Pro-OMe:

The dried compound 2.3 is dissolved with an appropriate amount of diethylamine, concentrated to a minimum amount, precipitated by addition of saturated NaHCO₃, filtered, washed to neutral with water, dried in vacuum, washed by stirring in petroleum ether three times, filtered, and dried in vacuum to give compound 2.4.

### (5)Synthesis of Compound 2.5: Fmoc-Gln(Trt)-Asn(Trt)-Cys(Acm)-Pro-OMe:

Fmoc-Gln(Trt)-OH (1 eq.) and HOBt (1.1 eq.) are dissolved in the mixture solvent of THF and a small amount of DMF, and then cooled to -10°C in an ice bath for 10 min. To the cool solution is added DCC (1.1 eq.) dissolved in a small amount of THF. After reaction for 20 minutes, compound 2.4 (1eq.), dissolved in THF, is added to the above reaction solution. The reaction is kept at 15 -25°C, monitored by HPLC, until it is completed. The mixture solution is filtrated with removal of insoluble matter, concentrated and subsequently separated solid out by addition of 0.1M HCl. The solid is then collected by filtration, washed with water until neutral, and then dried in vacuum to give compound 2.5.

### (6) Synthesis of Compound 2.6: H-Gln(Trt)-Asn(Trt)-Cys(Acm)-Pro-OMe:

The dried compound 2.5 is dissolved in an appropriate amount of diethylamine, concentrated to a minimum amount, precipitated by addition of saturated NaHCO₃, filtered, washed to neutral with water, dried in vacuum, washed by stirring in petroleum ether three times, filtered, and dried in vacuum to give compound 2.6.

### (7) Synthesis of compound 2.7: Fmoc-Ile-Gln(Trt)-Asn(Trt)-Cys(Acm)-Pro-OMe:

Fmoc-Ile-OH (1 eq.) and HOBt (1.1 eq.) are dissolved in the mixture solvent of THF and a small amount of DMF, and then cooled to -10°C in an ice bath for 10 min. To the cool solution is added DCC (1.1 eq.) dissolved in a small amount of THF. After reaction for 20 minutes, compound 2.6(1eq.), dissolved in THF, is added to the above reaction solution. The reaction is kept at 15 -25°C, monitored by HPLC, until it is completed. The mixture solution is filtrated with removal of insoluble matter, concentrated and subsequently separated solid out by addition of 0.1M HCl. The solid is then collected by filtration, washed with water until neutral, and then dried in vacuum to give compound 2.7.

### (8) Synthesis of fragment 2: H-Ile-Gln(Trt)-Asn(Trt)-Cys(Acm)-Pro-OMe:

The dried compound 2.7 is dissolved in an appropriate amount of diethylamine, concentrated to a minimum amount, precipitated by addition of saturated NaHCO₃, filtered, washed to neutral with water, dried in vacuum, washed by stirring in petroleum ether three times, filtered, and dried in vacuum to give fragment 2.

### 3.Synthesis of fragment 3: H-Leu-Gly-NH₂

Fmoc-Leu-OH (1 eq.) and BOP (1 eq.) are dissolved in DMF and added with DIPEA (1.2eq.). After 5 minutes, H-Gly-NH₂.HCl (1.1 eq.), ssolved in DMF, is added with TEA (1.1 eq.), and poured into the above reaction solution. After the TLC(DCM:MeOH:AcOH=100:6:1)monitored reaction is completed, the solid product is precipitated by addition of 0.1M HCl, washed with water until neutral, and then dried in vacuum to give product Fmoc-Ile-Gly-NH₂.HCl. The dried Fmoc-Ile-Gly-NH2.HCl is dissolved in an appropriate amount of diethylamine. After the reaction is completed, indicated by TLC(DCM:MeOH:AcOH=100:6:1), the reaction mixture is concentrated, precipitated by petroleum ether, filtered, washed with petroleum ether and dried in vacuum to give fragment 3.

### 4.Synthesis of compound 4:

Fragment 1 (1.1 eq.) and HOBt (1.1 eq.) are dissolved in the mixture solvent of THF and a small amount of DMF, and then cooled to -10°C in an ice bath for 10 min. To the cool solution is added DCC (1.1 eq.) dissolved in a small amount of THF. After reaction for 20 minutes, fragment 2(1eq.), dissolved in THF, is added to the above reaction solution. The reaction is kept at 15 -25°C, monitored by HPLC, until it is completed. The mixture solution is filtrated with removal of insoluble matter, concentrated and precipitated by addition of saturated NaHCO₃.The solid is then collected by filtration, washed with water until neutral, and then dried in vacuum to give compound 4.

### 5.Synthesis of compound 5:

Compound 4 (1 eq.) is dissolve in THF, cooled to -10°C in an ice bath for 10 min and the reaction solution is maintained in the ice bath while 2M LiOH (5 eq.) is added. After the reaction is completed, indicated by HPLC, the mixture solution is regulated to neutral by addition of 0.5M HCl, concentrated to precipitate a white solid which is then filtered, washed with water and dried in vacuo to give compound 5.

### 6. Synthesis of compound 6:

Compound 5 (1 eq.) and BOP (1 eq.) are dissolve in DMF and added with DIPEA (1.1 eq.). After 5 min, fragment 3 (1.1 eq.), dissolved in DMF, is added to the above reaction solution. After the reaction is completed, indicated by HPLC, a white solid is precipitated by addition of 0.1M HCl, filtered, washed with water until neutral and dried in vacuum to give compound 6.

### 7. Synthesis of compound 7:

Iodine (10 eq.), dissolve in DMF, is slowly added to compound 6 (1 eq.). After the reaction is completed, indicated by HPLC, a white solid is precipitated by addition of 0.1% sodium thiosulfate solution, filtered, washed with water and dried in vacuum to give compound 7.

### 8. Synthesis of crude oxytocin:

Compound 7 is dissolved in trifluoroacetic acid, concentrated to a minimum amount after 10 min, added with DCM and concentrated to a minimum amount (three times), and then added with ethyl acetate, refrigerated overnight, filtered, and dried in vacuum.

### 9. Purification of crude oxytocin:

Pretreatment of the crude peptide: 0.5 g crude peptide is dissolved in 10 ml NaH₂PO₄ solution (0.1 M), followed by addition of acetonitrile to make the concentration of acetonitrile reach 15% in the solution, and then filtered through a 0.45 µm microporous membrane.

Purification conditions and gradients:
Preparation column: DAC HB-50, Fuji silica gel 100 Å -10µm-C18
Detection wavelength: 220nm, flow rate: 50ml/min
mobile phase: A 0.1M NaH₂PO₄ solution, B 50% acetonitrile/A, 0-60min: 30%B-50%B

Liquid contained oxytocin with purity above 99% is collected, concentrated at 25°C, desalted by PS polymer, concentrated, and freeze-dried to obtain the pure product of oxytocin (the HPLC image is shown in the attached drawings). The pure product of oxytocin provided by the invention has a content of 98.1% and a biological value of 588IU/mg measured by the content determination method according to the European Pharmacopoeia, with the European Pharmacopoeia standards used as the standard materials.

The beneficial effects of the embodiments of the present invention:
In the embodiments of the present invention, a Boc-polypeptide synthesis and Fmoc -polypeptide synthesis combined method is provided, wherein, all the reactions are carried out under mild conditions without using the ammonia-sodium-method decapping reaction which is reported by literature referring to oxytocin liquid-phase synthesis at home and abroad. Further more, the liquid-phase synthesis method of oxytocin is first performed without highly toxic reagents and unsafe reaction conditions, thus greatly reducing the cost of oxytocin synthesis and providing reference for industrial production of oxytocin. Besides, high purity (above 99%) and high biological value (588 IU/mg) of oxytocin is also provided in the embodiments of the present invention.

Full names corresponding to the abbreviation of the substance appearing in the claims and description of the embodiments of the present invention are shown in Table 1.

**Table 1**

| Abbreviation | Full name |
|---|---|
| Fmoc | *9- fluorene methoxy carbonyl* |
| Trt | trityl |
| HOBt | I-hydroxybenzotriazole |
| Boc | t-Butyloxy carbonyl |
| tBu | tertiary butyl |
| TFA | trifluoroacetic acid |
| TIS | Triisopropylsilane |
| DIPEA | *n,n-diisopropylethylamine* |
| IPA | isopropanol |
| DMF | *N,N-dimethylformamide* |
| Gly | glycine |
| Leu | leucine |
| Pro | proline |
| Asn | L-Asparagine Monohydrate |
| Gln | glutamine |
| Cys | cysteine |
| Ile | isoleucine |
| Tyr | Tyrosine |
| THF | Tetrahydrofuran |
| DCC | Dicyclohexylcarbodiimide |
| HOOBt | 3,4-Dihydro-3-hydroxy-4-oxo-1,2,3-benzotriazine |
| TEA | triethylamine |
| DEA | diethylamine |
| HOSu | 1-hydroxy-5-pyrrolidinedione |

All the following Examples 1 to 10 are not seriously covered by the claims. However, they are highly related to some of the steps in the claims and therefore useful for understanding the present invention.

### Example 1: Synthesis of fragment 1: Boc-Cys(Acm)-Tyr(tBu)-OH

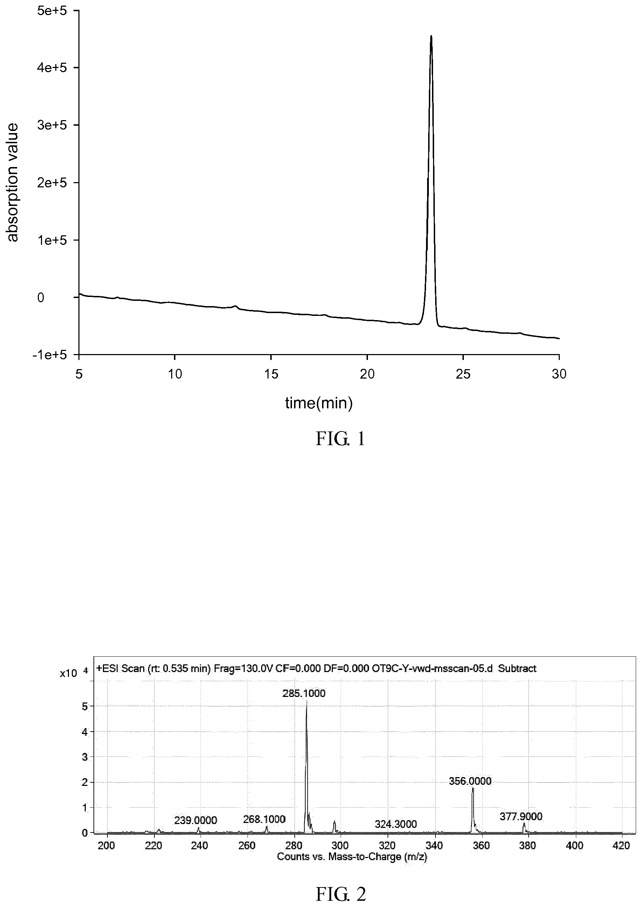

Boc-Cys(Acm)-OH (100 mol, 29.2 g, 1 eq.) and HOSu (110 mmol, 12.6 g, 1.1 eq.) were dissolved in 200mL THF, and then cooled to -10°C in an ice bath for 10 min. To the cool solution was added DCC (110 mmol, 22.7 g, 1.1eq.) dissolved in 20mL THF. After reaction for 20 minutes, H-Tyr(tBu)-OH (110 mmol, 26.1 g, 1.1 eq.) and NaHCO₃ (110 mmol, 9.2 g, 1.1 eq.) were dissolve in 150mL water and then added to the above solution. The reaction was kept at 15 -25°C, monitored by HPLC, until it was completed. The mixture solution was regulated to neutral by addition of 0.5M HCl, filtrated with removal of insoluble matter, concentrated to remove THF and subsequently regulated to pH=2 by addition of 0.5M HCl. The obtained white solid was then collected by filtration, washed with water until neutral, and then dried in vacuum to give 47.1 g fragment 1 with 99.1% purity and 92% yield. Fragment 1: Boc-Cys(Acm)-Tyr(tBu)-OH. Its RP-HPLC image is shown in Fig. 1 and its ESI-MS spectrum of strong-acid hydrolysis sample (H-Cys(Acm)-Tyr-OH m/z calculated.355.4; found 356.3 [M+H]⁺) is shown in FIG. 2

### Example 2 Synthesis of fragment 2: H-Ile-Gln(Trt)-Asn(Trt)-Cys(Acm)-Pro-OMe

### (1)Synthesis of compound 2.1: Fmoc-Cys(Acm)-Pro-OMe:

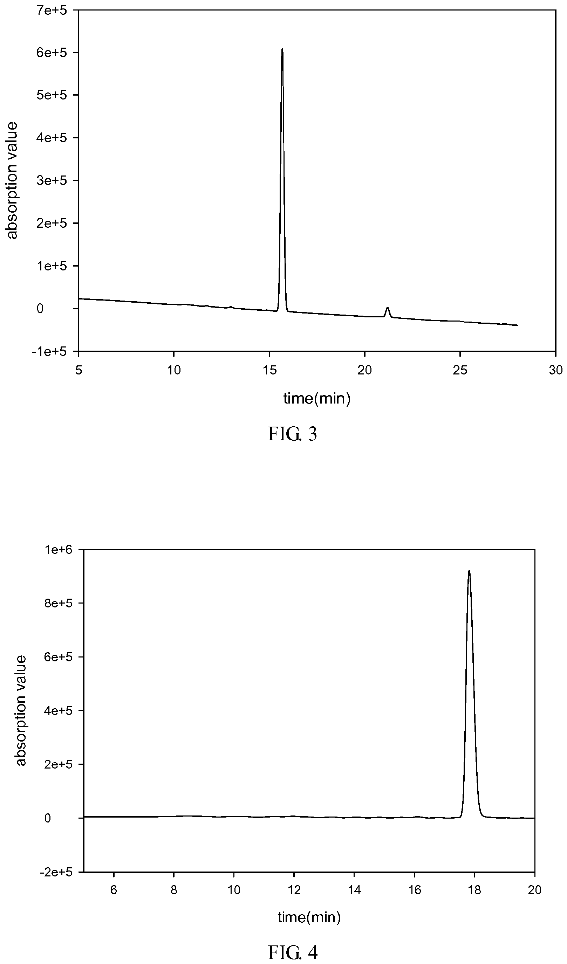

Fmoc-Cys(Acm)-OH (100 mmol, 41.5 g, 1 eq.) and HOBt (105 mmol, 14.1 g, 1.05 eq.) were dissolved in the mixture solvent of 200 mL THF and 30 mL DMF, and then cooled to -10°C in an ice bath for 10 min. To the cool solution was added DCC (105 mmol, 21.6g, 1.05 eq.) dissolved in 20 mL THF. After reaction for 20 minutes, H-Pro-OMe.HCl (105 mmol, 17.4 g, 1.05 eq.), dissolved in 100 mL THF and well-mixed with TEA (105 mmol, 14.5 ml, 1.05 eq.), was added to the above reaction solution. The reaction was kept at 15 -25°C, monitored by HPLC, until it was completed. The mixture solution was filtrated with removal of insoluble matter, concentrated and subsequently separated solid out by addition of 0.1M HCl. The solid was then collected by filtration, washed to neutral by water, and then dried in vacuum to give 50.9 g compound 2.1 with 95.3% purity and 97% yield (the yield is calculated in mmol, the same as below). The HPLC image is shown in Fig. 3.

### (2) Synthesis of compound 2.2: H-Cys(Acm)-Pro-OMe:

The dried compound 2.1 was dissolved in 200mL diethylamine, concentrated to a minimum amount, precipitated by addition of petroleum ether, filtered, washed with petroleum ether, and dried in vacuum to give 28.8 g compound 2.2 with 98.0% purity and 95% yield. The HPLC image is shown in Fig. 4.

### (3) Synthesis of compound 2.3: Fmoc-Asn(Trt)-Cys(Acm)-Pro-OMe:

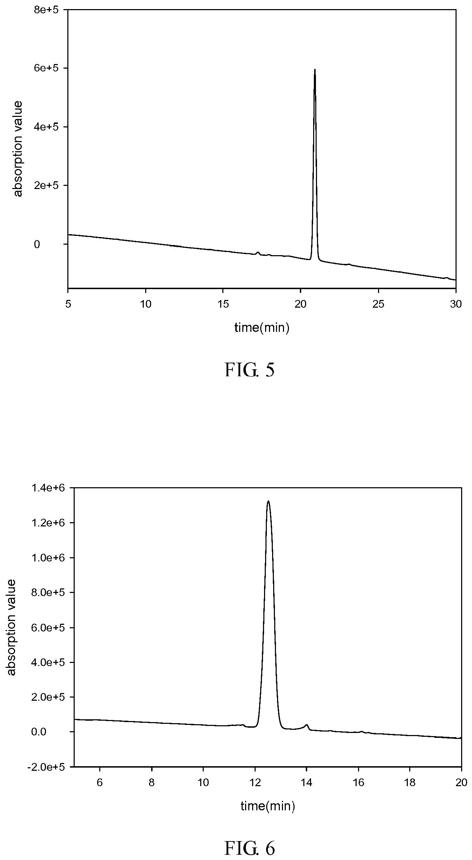

Fmoc-Asn(Trt)-OH (1.1 eq., 104.4 mmol, 62.3 g) and HOBt (1.1 eq., 104.4 mmol, 14.1 g) were dissolved in the mixture solvent of 200 mL DCM and 20 mL DMF, and then cooled to -10°C for 10 min. To the cool solution was added DCC (1.1 eq., 104.4 mmol, 21.5 g) dissolved in 20mL DCM. After reaction for 20 minutes, compound 2.2 (1 eq., 94.9 mmol, 28.8 g), dissolved in 50 mL DMF, was added to the above reaction solution. The reaction was kept at 15 -25°C, monitored by HPLC, until it was complete. The mixture solution was filtrated with removal of insoluble matter, concentrated and subsequently separated solid out by addition of 0.1M HCl. The solid was then collected by filtration, washed to neutral by water, and then dried in vacuum to give 75 g compound 2.3 with 99% purity and 85% yield. The HPLC image is shown in Fig. 5.

### (4) Synthesis of compound 2.4: H-Asn(Trt)-Cys(Acm)-Pro-OMe:

The dried compound 2.3 was dissolved with 200 mL diethylamine, concentrated to a minimum amount, precipitated by addition of saturated NaHCO₃, filtered, washed to neutral with water, dried in vacuum, washed by stirring in petroleum ether three times, filtered, and dried in vacuum to give 55.7 g compound 2.4 with 96.3% purity and 84.4% yield. The HPLC image is shown in Fig. 6.

### (5) Synthesis of compound 2.5: Fmoc-Gln(Trt)-Asn(Trt)-Cys(Acm)-Pro-OMe:

Fmoc-Gln(Trt)-OH (1.1 eq., 92.8 mmol, 56.7 g) and HOBt (1.1 eq., 92.8 mmol, 12.5 g) were dissolved in the mixture solvent of 150 mL THF and 50 mL DMF, and then cooled to -10°C for 10 min. To the cool solution was added DCC (1.1 eq., 92.8 mmol, 19.1 g) dissolved in 20 mL THF. After reaction for 20 minutes, compound 2.4 (1 eq., 84.4 mmol, 55.7 g), dissolved in 150 mL THF, was added to the above reaction solution. The reaction was kept at 15 -25°C, monitored by HPLC, until it was complete. The mixture solution was filtrated with removal of insoluble matter, concentrated and subsequently separated solid out by addition of 0.1M HCl. The solid was then collected by filtration, washed to neutral by water, and then dried in vacuum to give 106.4 g compound 2.5 with 85% yield.

### (6) Synthesis of compound 2.6: H-Gln(Trt)-Asn(Trt)-Cys(Acm)-Pro-OMe:

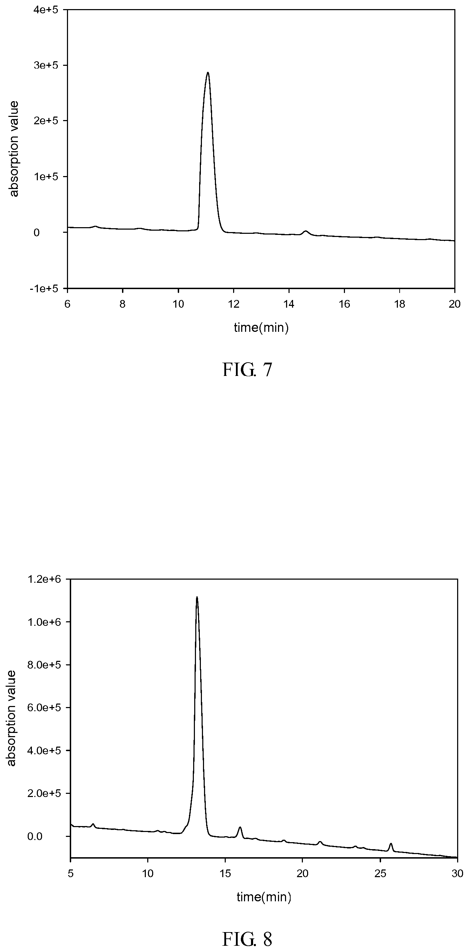

The dried compound 2.5 was dissolved in 150 mL diethylamine, concentrated to a minimum amount, precipitated by addition of saturated NaHCO₃, filtered, washed to neutral with water, dried in vacuum, washed by stirring in petroleum ether three times, filtered, and dried in vacuum to give 86.5 g compound 2.6 with 98.3. % purity and 84% yield. The HPLC image is shown in Fig. 7.

### (7) Synthesis of compound 2.7: Fmoc-Ile-Gln(Trt)-Asn(Trt)-Cys(Acm)-Pro-OMe:

Fmoc-Ile-OH (1.1 eq., 92.4 mmol, 32.6 g) and HOBt (1.1 eq., 92.4 mmol, 12.5 g) were dissolved in the mixture solvent of 150 mL THF and 50 mL DMF, and then cooled to -10°C for 10 min. To the cool solution was added DCC (1.1 eq., 92.4 mmol, 19 g) dissolved in 20 mLTHF. After reaction for 20 minutes, compound 2.6(1 eq., 84 mmol, 86.5 g), dissolved in 150 mL THF, was added to the above reaction solution. The reaction was kept at 15 -25°C, monitored by HPLC, until it was complete. The mixture solution was filtrated with removal of insoluble matter, concentrated and subsequently separated solid out by addition of 0.1M HCl. The solid was then collected by filtration, washed to neutral by water, and then dried in vacuum to give 114.4 g compound 2.7 with 90.3% purity and 83.8% yield.

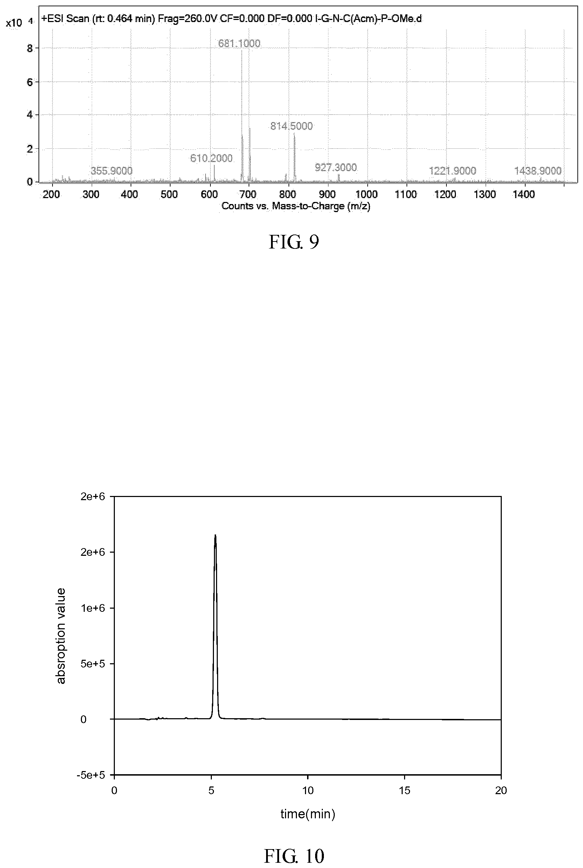

(8) Synthesis of fragment 2: H-Ile-Gln(Trt)-Asn(Trt)-Cys(Acm)-Pro-OMe: he dried compound 2.7 was dissolved in 150 mL diethylamine, concentrated to a minimum amount, precipitated by addition of saturated NaHCO₃, filtered, washed to neutral with water, dried in vacuum, washed by stirring in petroleum ether three times, filtered, and dried in vacuum to give 91.5 g fragment 2 with 80.1% yield. The HPLC image is shown in Fig. 8 and the ESI-MS spectrum of its strong-acid hydrolysis sample (H-Ile-Gln-Asn-Cys(Acm)-Pro-OMe m/z calculated.658.3; found 681.1.3 [M+Na]⁺) is shown in Fig. 9.

### Example 3:

### Synthesis of fragment 3 according to solution 2: H-Leu-Gly-NH₂:

Fmoc-Leu-OH (1 eq., 100 mmol, 35.3 g) and BOP (1.01 eq., 101 mmol, 44.7 g) were dissolved in 200 mL DMF and added with DIPEA (1.2 eq., 120 mmol, 21 ml). After 5 minutes, H-Gly-NH₂. HCl (1.1 eq., 110 mmol, 12.2 g), dissolved in 100 mL DMF, was added with TEA (1.1 eq., 110 mmol, 15 ml), and poured into the above reaction solution. After the TLC (DCM:MeOH:AcOH=100:6:1) monitored reaction was completed, the solid product was precipitated by addition of 0.1M HCl, washed with water until neutral, and then dried in vacuum to give 43.7 g product Fmoc-Ile-Gly-NH₂. HCl with 97% purity and 98% yield.

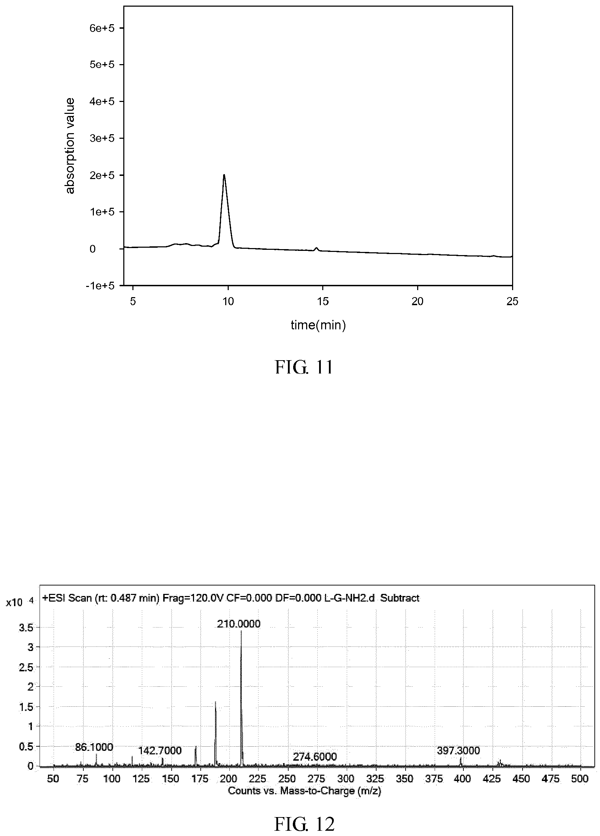

The dried Fmoc-Ile-Gly-NH2.HCl was dissolved in 400 mLdiethylamine. After the reaction was completed, indicated by TLC (DCM:MeOH:AcOH=100:6:1), the reaction mixture was concentrated, precipitated by petroleum ether, filtered, washed with petroleum ether and dried in vacuum to give 17.9 g fragment 3 with 98.4% purity and 95.6% yield. The HPLC image is shown in Fig.11 and the ESI-MS spectrum is shown in Fig. 12 (H-Leu-Gly-NH₂ m/z calculated. 187.1; found 210.0[M+Na]⁺).

### Example 4:

### Synthesis of compound 4:

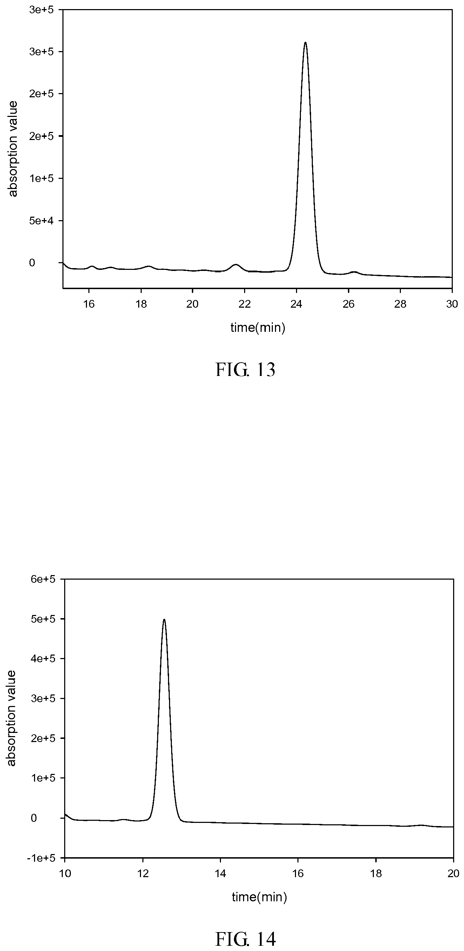

Fragment 1 (1.1 eq., 88 mmol, 45 g) and HOBt ((1.1 eq., 88 mmol, 11.9 mg) were dissolved in the mixture solvent of 150 mL THF and 60 mL DMF, and then cooled to -10°C in an ice bath for 10 min. To the cool solution was added DCC (1.1 eq., 88 mmol, 18.1 mg) dissolved in 20 mL THF. After reaction for 20 minutes, fragment 2(1 eq., 80 mmol, 91.5 g), dissolved in 100 mL THF, was added to the above reaction solution. The reaction was kept at 15 -25°C, monitored by HPLC, until it was completed. The mixture solution was filtrated with removal of insoluble matter, concentrated and precipitated by addition of saturated NaHCO₃.The solid was then collected by filtration, washed with water until neutral, and then dried in vacuum to give 115.5 g compound 4 with 86% purity and 70.6% yield. The RP-HPLC image is shown in Fig. 13.

### Example 5:

### Synthesis of compound 5:

Compound 4 (1 eq., 115.5 g) was dissolve in 800 mL THF, cooled to -10°C in an ice bath for 10 min and the reaction solution was maintained in the ice bath while 1.5M NaOH (5 eq., 353 mmol, 235mL) was added. After the reaction was completed, indicated by HPLC, the mixture solution was regulated to neutral by addition of 0.5M HCl, concentrated to precipitate a white solid which is then filtered, washed with water and dried in vacuum to give 106.2 g compound 5 with 93% purity and 65.5% yield. The RP-HPLC image is shown in Fig. 14.

### Example 6:

### Synthesis of compound 6:

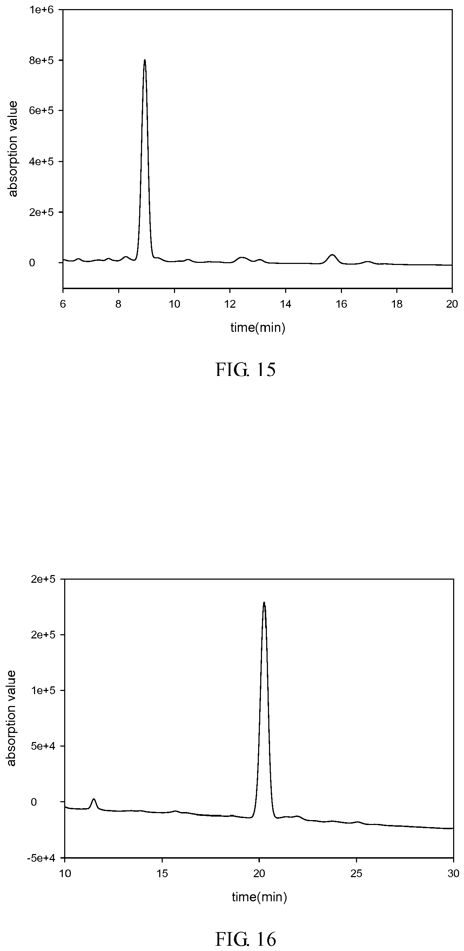

Compound 5 (1 eq., 65.5 mmol, 106.2 g) and BOP (1.05 eq., 68.8 mmol, 30.4 g) were dissolve in 150 mL DMF and added with DIPEA (1.1 eq., 72 mmol, 12.5 ml). After 5 min, fragment 3 (1.1 eq., 72 mmol, 12.5 ml), dissolved in 100 mL DMF, was added to the above reaction solution. After the reaction was completed, indicated by HPLC, a white solid was precipitated by addition of 0.1M HCl, filtered, washed with water until neutral and dried in vacuum to give 107.5 g compound 6 with 88.6% purity and 60% yield. The RP-HPLC image is shown in Fig. 15.

### Example 7:

### Synthesis of compound 7:

Iodine (10 eq., 600 mmol, 152 g), dissolve in 120 mL DMF, was slowly added to compound 6 (1 eq., 60 mmol, 107.5 g). After the reaction was completed, indicated by HPLC, a white solid is precipitated by addition of 0.1% sodium thiosulfate solution, filtered, washed with water and dried in vacuum to give 96.5 g compound 7 with 95% purity and 58.6% yield. The RP-HPLC image is shown in Fig. 16.

### Example 8:

### Synthesis of crude oxytocin:

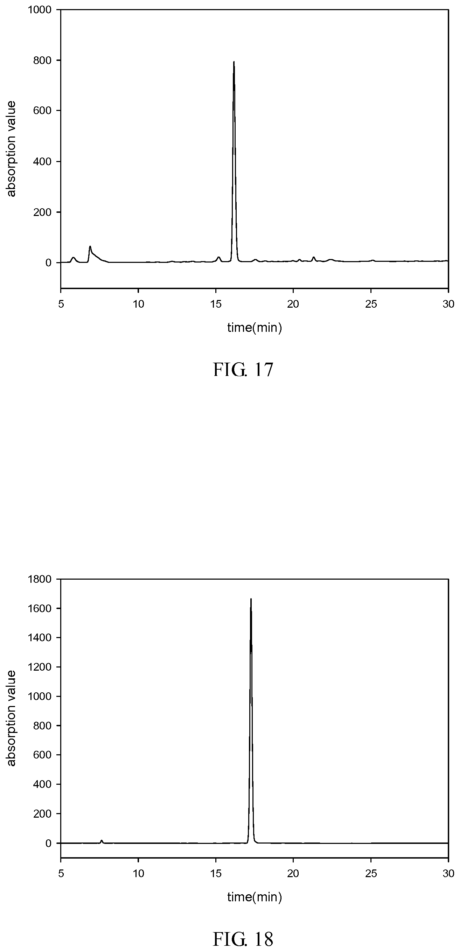

Compound 7 was dissolved in 200 mL trifluoroacetic acid hydrolysis reagent (TFA:TIS:H₂O=95:5:5), concentrated to a minimum amount after 10 min, added with DCM and concentrated to a minimum amount (three times), and then added with ethyl acetate, refrigerated for 10h, filtered, and dried in vacuum to obtain 26.6 g crude oxytocin with 94.5% purity and 26.4% yield. The RP-HPLC image is shown in Fig. 17.

### Example 9:

Pretreatment of the crude peptide: 0.5 g crude peptide was dissolved in 10 ml NaH₂PO₄ solution (0.1 M), followed by addition of acetonitrile to make the concentration of acetonitrile reach 15% in the solution, and then filtered through a 0.45 µm microporous membrane.

Purification conditions and gradients:
Preparation column: DAC HB-50, Fuji silica gel 100 Å -10µm-C18
Detection wavelength: 220nm, flow rate: 50ml/min
Mobile phase: A 0.1M NaH₂PO₄ solution, B 50% acetonitrile/A, 0-60min: 30%B-50%B

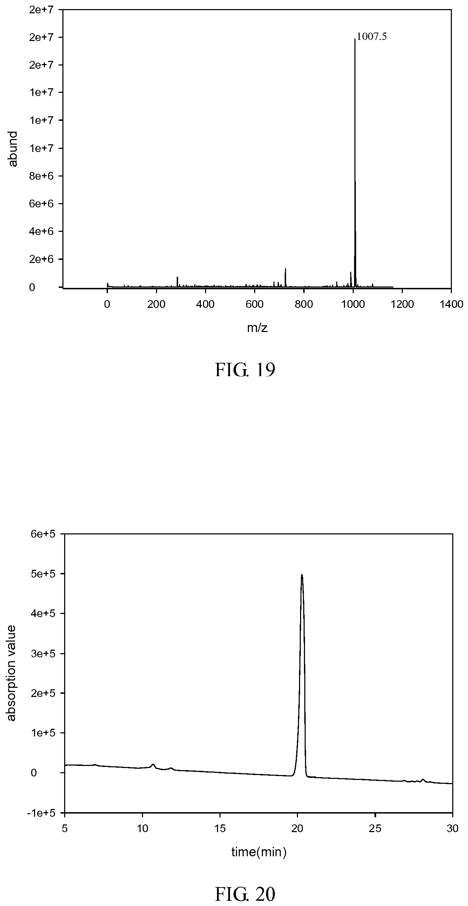

Liquid contained oxytocin with purity above 99% was collected, concentrated at 25°C, desalted by PS polymer, added with acetic acid, concentrated, and freeze-dried to obtain 10.6 g oxytocin (the HPLC image is shown in Fig. 18 and the ESI-MS spectrum is shown in Fig. 19) with a content of 98.2% and a biological value of 588IU/mg, while water and acetic acid was removed, measured by the content determination method according to the European Pharmacopoeia, with the European Pharmacopoeia standards used as the standard materials.

### Example 10

### Synthesis of Fragment 3 according to solution 1:

Boc-Leu-OH (1 eq., 100 mmol, 23.1 g) and BOP (1.01 eq., 101 mmol, 44.7 g) were dissolved in 200 ml DCM and 20 ml DMF, and added with DIPEA (1.2 eq., 120 mmol, 21 ml) .After 5 min, H-Gly-NH₂. HCl (1.1 eq., 110 mmol, 12.2 g), dissolved in 100 ml DMF and added with TEA (1.1 eq., 110 mmol, 15 ml), was poured to the above reaction mixture, traced by TLC analysis(DCM:MeOH:AcOH=100:20:1). After the reaction was completed, the white thick material was precipitated with 0.5 M HCl solution, dissolved in EA, extracted, washed with water three times, washed with saturated NaCl once, dried by anhydrous MgSO₄ for 30 min, filtered, and concentrated to obtain dry product with 99.1% purity. The HPLC image is shown in Fig. 20.

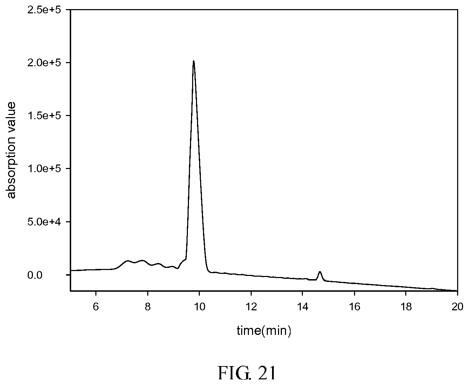

The obtained product was added with 200 ml of 30% TFA/DCM (trifluoroacetic acid/dichloromethane) solution, after 30 minutes reaction, concentrated to dryness, and dried in vacuum to obtain 25.6 g product H-Leu-Gly-NH₂ with 96% purity, and 85% yield. The HPLC image is shown in FIG. 21.

## Claims

1. A method for manufacturing oxytocin by liquid-phase polypeptide synthesis, which comprises the following proceedings:
(1) synthesizing fragment 1: Boc-Cys(Acm)-Tyr(tBu)-OH;
(2) synthesizing fragment 2: H- Ile-Gln(Trt)-Asn(Trt)-Cys(Acm)-Pro-OMe;
(3) synthesizing fragment 3: H-Leu-Gly-NH₂;
(4) synthesizing compound 4: Boc-Cys(Acm)-Tyr(tBu)-Ile-Gln(Trt)-Asn(Trt)-Cys(Acm)-Pro-OMe;
(5) synthesizing compound 5: Boc-Cys(Acm)-Tyr(tBu)-Ile-Gln(Trt)-Asn(Trt)-Cys(Acm)-Pro-OH;
(6) synthesizing compound 6: Boc-Cys(Acm)-Tyr(tBu)-Ile-Gln(Trt)-Asn(Trt)-Cys(Acm)-Pro-Leu-Gly-NH₂;
(7) synthesizing compound 7: Boc-Cys-Tyr(tBu)-Ile-Gln(Trt)-Asn(Trt)-Cys-Pro-Leu-Gly-NH2 by using iodine as a reagent;
(8) deprotecting compound 7 with trifluoroacetic acid and concentrating the reaction mixture to obtain a crude oxytocin solution;
(9) purifying the crude product by C18 reversed-phase silica gel chromatography to obtain pure oxytocin;
wherein synthesizing the fragment 2 comprises the following proceedings:
(1) synthesizing compound 2.1: Fmoc-Cys(Acm)-Pro-OMe:
a ratio of an amount of Fmoc-Cys(Acm)-OH to an amount of H-Pro-OMe.HCl is from 1:1.05 to 1:1.5; DIC or DCC is employed as a condensing agent; a ratio of an amount of the condensing agent to the amount of H-Pro-OMe.HCl is 1:1; HOSu or HOBt or HOOBt is employed as an activator; a ratio of an amount of the activator to the amount of H-Pro-OMe.HCl is 1:1; triethylamine (TEA) diisopropylethylamine (DIPEA) or N-methylmorpholine (NMM) is employed as a base; a ratio of an amount of the base to the amount of H-Pro-OMe.HCl is 1:1; and DMF, DMSO, DCM or THF is employed as a solvent;
(2) synthesizing compound 2.2: H-Cys(Acm)-Pro-OMe:
deprotecting the compound 2.1 with diethylamine (DEA) or 5% piperazine/DCM as a deprotecting reagent, concentrating after completion of the deprotecting reaction, using petroleum ether to separate the product, filtrating and vacuum-drying to give compound 2.2;
(3) synthesizing ompound 2.3: Fmoc -Asn(Trt)-Cys(Acm)-Pro-OMe:
a ratio of an amount of the compound 2.2 to an amount of Fmoc-Asn(Trt)-OH is from 1:1.05 to 1:1.5; DIC or DCC is employed as a condensing agent, a ratio of an amount of the condensing agent to the amount of Fmoc-Asn(Trt)-OH is 1:1; HOSu, HOOBt or HOBt is employed as an activator; a ratio of an amount of the activator to the amount of Fmoc-Asn(Trt)-OH is 1:1; and DMF, DMSO, DCM, or THF is employed as a solvent;
(4) synthesizing compound 2.4: H-Asn(Trt)-Cys(Acm)-Pro-OMe:
deprotecting the compound 2.3 with diethylamine (DEA) or 5% piperazine/DCM as a deprotecting reagent; concentrating after completion of the deprotecting reaction, using saturated NaHCO₃ solution to separate the product, filtrating, washing with water to neutral and drying in vacuum, and then washing a crude product with petroleum ether while stirring, filtrating and drying in vacuum to give compound 2.4;
(5) synthesizing compound 2.5: Fmoc-Gln(Trt)-Asn(Trt)-Cys(Acm)-Pro-OMe:
a ratio of an amount of the compound 2.4 to an amount of Fmoc-Gln(Trt)-OH is from 1:1.05 to 1:1.5; DIC or DCC is employed as a condensing agent; a ratio of an amount of the condensing agent to the amount of Fmoc-Gln(Trt)-OH is 1:1; HOSu, HOOBt or HOBt is employed as an activator, a ratio of an amount of the activator to the amount of Fmoc-Gln(Trt)-OH is 1:1; and DMF, DMSO, DCM or THF is employed as a solvent;
(6) synthesizing compound 2.6: H-Gln(Trt)-Asn(Trt)-Cys(Acm)-Pro-OMe:
deprotecting the compound 2.5 with diethylamine (DEA) or 5% piperazine/DCM as a deprotecting reagent; concentrating after completion of the deprotecting reaction, using saturated NaHCO₃ solution to separate a product, filtrating, washing with water to neutral and drying in vacuum, and then washing a crude product with petroleum ether while stirring, filtrating and drying in vacuum to give compound 2.6;
(7) synthesizing compound 2.7: Fmoc-Ile-Gln (Trt)-Asn(Trt)-Cys(Acm)-Pro-OMe:
a ratio of an amount of compound 2.6 to an amount of Fmoc-Ile-OH is 1:1.05-1:1.5; DIC or DCC is employed as a condensing agent; a ratio of an amount of the condensing agent to an amount of Fmoc-Ile-OH is 1:1; HOSu, HOOBt or HOBt is employed as an activator; a ratio of an amount of the activator to the amount of Fmoc-Ile-OH is 1:1; and DMF, DMSO, DCM or THF is employed as a solvent;
(8) synthesizing fragment 2: H-Ile-Gln(Trt)-Asn(Trt)-Cys(Acm)-Pro-OMe:
deprotecting the compound 2.7 with diethylamine (DEA) or 5% piperazine/DCM as a deprotecting reagent; concentrating after completion of the deprotecting reaction, using saturated NaHCO₃ solution to separate a product, filtrating, washing with water to neutral and drying in vacuum, and then washing a crude product with petroleum ether while stirring, filtrating and drying in vacuum to give the fragment 2.

2. The method for manufacturing oxytocin by liquid-phase polypeptide synthesis according to claim 1, wherein, the fragment 1 in the proceeding (1) is: Boc-Cys(Acm)-Tyr(tBu)-OH; amino acid units for synthesizing the fragment 1 are Boc-Cys(Acm)-OH and H-Tyr(tBu)-OH; DIC or DCC is employed as a condensing agent; HOSu or HOBt is employed as an activator; THF and water are employed as a solvent; and the ratio of the THF to the water is from 1:1 to 5:1, wherein the solvent may also be DMF or DMSO.

3. The method for manufacturing oxytocin by liquid-phase polypeptide synthesis according to claim 1, wherein the fragment 3 in the proceeding (3) is: H-Leu-Gly-NH₂; and the fragment 3 may be synthesized through solution 1 or solution 2 as follows:
solution 1: Boc-Leu-OH and H-Gly-NH₂.HCl are employed as amino acid units; BOP, HBTU, TBTU, DIC or DCC/HOBt or HOSu is employed as a condensing agent; TEA, NMM or DIPEA is employed as a base; DMF, DMSO, THF or DCM is employed as a solvent; synthesizing Boc-Leu-Gly-NH₂ and then removing the boc protecting group of it with TFA to obtain H-Leu-Gly-NH₂;
solution 2: Fmoc-Leu-OH, H-Gly-NH2.HCl are employed as amino acid units; BOP, HBTU, TBTU, DIC or DCC/HOBt or HOSu is employed as a condensing agent; TEA, NMM or DIPEA is employed as a base; DMF, DMSO, THF or DCM is employed as a solvent; after synthesizing Fmoc-Leu-Gly-NH₂, removing the Fmoc protecting group of it to obtain H-Leu-Gly-NH₂; and a deprotecting agent is diethylamine (DEA) or 5% piperazine/DCM.

4. The method for manufacturing oxytocin by liquid-phase polypeptide synthesis according to claim 1, wherein the compound 4 in the proceeding (4) is: Boc-Cys(Acm)-Tyr(tBu)-Ile-Gln(Trt)-Asn(Trt)-Cys(Acm)-Pro-OMe and the synthesis of compound 4 comprises the following proceedings:
a ratio of an amount of the fragment 1 to an amount of the fragment 2 is 1.05-1.5:1; BOP, DCC, DIC or an azide reagent is employed as a condensing agent; a ratio of an amount of the condensing agent to an amount of the fragment 1 is 1:1; HOSu or HOBt is employed as an activator; a ratio of an amount of the activator to the fragment 1 is 1:1; and DCM, THF , DMF or DMSO is employed as a solvent.

5. The method for manufacturing oxytocin by liquid-phase polypeptide synthesis according to claim 1, wherein the compound 5 in the proceeding (5) is: Boc-Cys(Acm)-Tyr(tBu)-Ile-Gln(Trt)-Asn(Trt)-Cys(Acm)-Pro-OH and the synthesis of compound 5 comprises the following proceedings:
LiOH or NaOH is employed as a saponification reagent, an amount of which is 5-15 equivalents of the compound 4; a concentration is 0.05-0.2 M; and THF, DMF, methanol, acetonitrile or DMSO is employed as a solvent.

6. The method for manufacturing oxytocin by liquid-phase polypeptide synthesis according to claim 1, wherein the compound 6 in the proceeding (6) is: Boc-Cys(Acm)-Tyr(tBu)-Ile-Gln(Trt)-Asn(Trt)-Cys(Acm)-Pro-Leu-Gly-NH₂; and the synthesis of compound 6 comprises the following proceedings:
a ratio of an amount of the compound 5 to an amount of the fragment 3 is 1:1:1.05-2; DCC, DIC, BOP or an azide reagent is employed as a condensing agent; HOSu, HOOBt or HOBt is employed as an activator; and THF, DMF or DMSO is employed as a solvent.

7. The method for manufacturing oxytocin by liquid-phase polypeptide synthesis according to claim 1, wherein in the proceeding (7), synthesizing compound 7 using iodine as a reagent, an amount of the iodine is 5-10 equivalents of the compound 6 and a concentration of the iodine is 0.1-0.5M, and DMF is employed as a solvent; after completion of the reaction, using 0.1% sodium thiosulfate solution to separate the solid, washing with water and drying in vacuum to give the compound 7.

## Patentansprüche

1. Verfahren zur Herstellung von Oxytocin durch Flüssigphasen-Polypeptidsynthese, das die folgenden Schritte aufweist:
(1) Synthetisieren von Fragment 1: Boc-Cys(Acm)-Tyr(tBu)-OH;
(2) Synthetisieren von Fragment 2: H-Ile-Gln(Trt)-Asn(Trt)-Cys(Acm)-Pro-OMe:
(3) Synthetisieren von Fragment 3: H-Leu-Gly-NH₂;
(4) Synthetisieren von Verbindung 4: Boc-Cys(Acm)-Tyr(tBu)-Ile-Gln(Trt)-Asn(Trt)-Cys(Acm)-Pro-OMe;
(5) Synthetisieren von Verbindung 5: Boc-Cys(Acm)-Tyr(tBu)-Ile-Gln(Trt)-Asn(Trt)-Cys(Acm)-Pro-OH;
(6) Synthetisieren von Verbindung 6: Boc-Cys(Acm)-Tyr(tBu)-Ile-Gln(Trt)-Asn(Trt)-Cys(Acm)-Pro-Leu-Gly-NH₂;
(7) Synthetisieren von Verbindung 7: 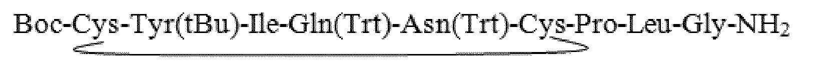
Boc-Cys-Tyr(tBu)-Ile-Gln(Trt)-Asn(Trt)-Cys-Pro-Leu-Gly-NH2
 unter Verwendung von Jod als Reagenz;
(8) Aufreinigen von Verbindung 7 mit Trifluoressigsäure und Konzentrieren der Reaktionsmischung, um eine rohe Oxytocin-Lösung zu erhalten;
(9) Reinigen des Rohprodukts durch C18-Umkehrphsen-Kieselgel-Chromatographie, um reines Oxytocin zu erhalten;
wobei das Synthetisieren des Fragmentes 2 die folgenden Schritte umfasst:
(1) Synthetisieren von Verbindung 2.1: Fmoc-Cys(Acm)-Pro-OMe;
ein Verhältnis der Menge von Fmoc-Cys(Acm)-OH zu einer Menge von H-Pro-OMe.HCl beträgt von 1 : 1,05 bis 1 : 1.5; DIC oder DCC wird als Kondensationsmittel eingesetzt; ein Verhältnis der Menge des Kondensationsmittels zu der Menge von H-Pro-OMe.HCl liegt bei 1 : 1; HOSu oder HOBt oder HOOBt wird als Aktivierungsmittel eingesetzt; ein Verhältnis der Menge des Aktivierungsmittels zu der Menge von H-Pro-OMe.HCl liegt bei 1 : 1; Triethylamin (TEA), Diisopropylethylamin (DIPEA) oder N-methylmorpholin (NMM) wird als Base eingesetzt; ein Verhältnis der Menge der Base zu der Menge von H-Pro-OMe.HCl liegt bei 1 : 1; und DMF, DMSO, DCM oder THF wird als ein Lösemittel eingesetzt;
(2) Synthetisieren von Verbindung 2.2: H-Cys(Acm)-Pro-OMe;
Aufreinigen der Verbindung 2.1 mit Diethylamin (DEA) oder 5%-igem Piperazin/DCM als Aufreinigungsreagenz, Konzentrierung nach Beendigung der Aufreinigungsreaktion, Verwendung von Waschbenzin zum Abtrennen des Produktes, Filtration und Vakuumtrocknen, um die Verbindung 2.2 zu erhalten;
(3) Synthetisieren von Verbindung 2.3: Fmoc-Asn(trt)-Cys(Acm)-Pro-OMe;
ein Verhältnis der Menge der Verbindung 2.2 zu der Menge von Fmoc-Asn(Trt)-OH beträgt von 1 : 1,05 bis 1 : 1.5; DIC oder DCC wird als Kondensationsmittel eingesetzt; ein Verhältnis der Menge des Kondensationsmittels zu der Menge von Fmoc-Asn(Trt)-OH liegt bei 1 : 1; HOSu, HOOBt oder HOBt wird als Aktivierungsmittel eingesetzt; ein Verhältnis der Menge des Aktivierungsmittels zu der Menge von Fmoc-Asn(Trt)-OH liegt bei 1 : 1; und DMF, DMSO, DCM oder THF wird als ein Lösemittel eingesetzt;
(4) Synthetisieren von Verbindung 2.4: H-Asn(Trt)-Cys(Acm)-Pro-OMe;
Aufreinigen der Verbindung 2.3 mit Diethylamin (DEA) oder 5%-igem Piperazin/DCM als Aufreinigungsreagenz, Konzentrierung nach Beendigung der Aufreinigungsreaktion, Verwendung einer gesättigten NaHCO₃-Lösung zum Abtrennen des Produktes, Filtration, Waschen mit Wasser zur Neutralisation und Vakuumtrocknen, und dann Waschen eines Rohproduktes mit Waschbenzin unter Rühren, Filtrieren und Vakuumtrocknen, um die Verbindung 2.4 zu erhalten;
(5) Synthetisieren von Verbindung 2.5: Fmoc-Gln(Trt)-Asn(Trt)-Cys(Acm)-Pro-OMe;
ein Verhältnis der Menge der Verbindung 2.4 zu der Menge von Fmoc-Gln(Trt)-OH beträgt von 1 : 1,05 bis 1 : 1.5; DIC oder DCC wird als Kondensationsmittel eingesetzt; ein Verhältnis der Menge des Kondensationsmittels zu der Menge von Fmoc-Gln(Trt)-OH liegt bei 1 : 1; HOSu, HOOBt oder HOBt wird als Aktivierungsmittel eingesetzt; ein Verhältnis der Menge des Aktivierungsmittels zu der Menge von Fmoc-Gln(Trt)-OH liegt bei 1 : 1; und DMF, DMSO, DCM oder THF wird als ein Lösemittel eingesetzt;
(6) Synthetisieren von Verbindung 2.6: H-Gln(Trt)-Asn(Trt)-Cys(Acm)-Pro-OMe;
Aufreinigen der Verbindung 2.5 mit Diethylamin (DEA) oder 5%-igem Piperazin/DCM als Aufreinigungsreagenz, Konzentrierung nach Beendigung der Aufreinigungsreaktion, Verwendung einer gesättigten NaHCO₃-Lösung zum Abtrennen eines Produktes, Filtration, Waschen mit Wasser zur Neutralisation und Vakuumtrocknen, und dann Waschen eines Rohproduktes mit Waschbenzin unter Rühren, Filtrieren und Vakuumtrocknen, um die Verbindung 2.6 zu erhalten;
(7) Synthetisieren von Verbindung 2.7: Fmoc-Ile-Gln(Trt)-Asn(Trt)-Cys(Acm)-Pro-OMe;
ein Verhältnis der Menge der Verbindung 2.6 zu der Menge von Fmoc-Ile-OH beträgt von 1 1,05 bis 1 1.5; DIC oder DCC wird als Kondensationsmittel eingesetzt; ein Verhältnis der Menge des Kondensationsmittels zu der Menge von Fmoc-Ile-OH liegt bei 1 : 1; HOSu, HOOBt oder HOBt wird als Aktivierungsmittel eingesetzt; ein Verhältnis der Menge des Aktivierungsmittels zu der Menge von Fmoc-Ile-OH liegt bei 1 : 1; und DMF, DMSO, DCM oder THF wird als ein Lösemittel eingesetzt;
(8) Synthetisieren des Fragments 2: H-Ile-Gln(Trt)-Asn(Trt)-Cys(Acm)-Pro-OMe;
Aufreinigen der Verbindung 2.7 mit Diethylamin (DEA) oder 5%-igem Piperazin/DCM als Aufreinigungsreagenz, Konzentrierung nach Beendigung der Aufreinigungsreaktion, Verwendung einer gesättigten NaHCO₃-Lösung zum Abtrennen eines Produktes, Filtration, Waschen mit Wasser zur Neutralisation und Vakuumtrocknen, und dann Waschen eines Rohproduktes mit Waschbenzin unter Rühren, Filtrieren und Vakuumtrocknen, um das Fragment 2 zu erhalten.

2. Verfahren zur Herstellung von Oxytocin durch Flüssigphasen-Polypeptidsynthese gemäß Anspruch 1, wobei das Fragment 1 in Schritt (1) Boc-Cys(Acm)-Tyr(tBu)-OH ist; die Aminosäureeinheiten zum Synthetisieren des Fragmentes 1 Boc-Cys-(Acm)-OH und H-Tyr(tBu)-OH sind; DIC oder DCC als ein Kondensationsmittel eingesetzt wird; HOSu oder HOBt als ein Aktivierungsmittel eingesetzt wird; THF und Wasser als Lösemittel eingesetzt werden; und das Verhältnis von THF zu Wasser von 1 : 1 bis 5 : 1 beträgt, wobei das Lösemittel auch DMF oder DMSO sein kann.

3. Verfahren zur Herstellung von Oxytocin durch Flüssigphasen-Polypeptidsynthese gemäß Anspruch 1, wobei das Fragment 3 in Schritt (3) H-Leu-Gly-NH₂ ist; und wobei das Fragment 3 mittels Lösung 1 oder Lösung 2 wie folgt synthetisiert werden kann:
Lösung 1: Boc-Leu-OH und H-Gly-NH₂.HCl werden als Aminosäureeinheiten eingesetzt; BOP, HBTU, TBTU, DIC oder DCC/HOBt oder HOSu wird als Kondensationsmittel eingesetzt; TEA, NMM oder DIPEA wird als Base eingesetzt; DMF, DMSO, THF oder DCM wird als Lösemittel eingesetzt; Synthetisieren von Boc-Leu-Gly-NH₂ und dann Entfernen der boc-Schutzgruppe davon mit TFA, um H-Leu-Gly-NH₂ zu erhalten;
Lösung 2: Fmoc-Leu-OH, H-Gly-NH₂.HCl werden als Aminosäureeinheiten eingesetzt; BOP, HBTU, TBTU, DIC oder DCC/HOBt oder HOSu wird als Kondensationsmittel eingesetzt; TEA, NMM oder DIPEA wird als Base eingesetzt; DMF, DMSO, THF oder DCM wird als Lösemittel eingesetzt; nach dem Synthetisieren von Fmoc-Leu-Gly-NH₂, Entfernen der Fmoc-Schutzgruppe davon, um H-Leu-Gly-NH₂ zu erhalten; und ein Aufreinigungsmittel ist Diethylamin (DEA) oder 5%-iges Piperazin/DCM.

4. Verfahren zur Herstellung von Oxytocin durch Flüssigphasen-Polypeptidsynthese gemäß Anspruch 1, wobei die Verbindung 4 in Schritt (4) Boc-Cys(Acm)-Tyr(tBu)-Ile-Gln(Trt)-Asn(Trt)-Cys(Acm)-Pro-OMe ist und die Synthese der Verbindung 4 die folgenden Schritte umfasst:
ein Verhältnis der Menge des Fragmentes 1 zu der Menge des Fragmentes 2 beträgt 1,05 - 1,5 : 1; BOP, DCC, DIC oder ein Azidreagenz wird als Kondensationsmittel eingesetzt; ein Verhältnis der Menge des Kondensationsmittels zu der Menge des Fragmentes 1 beträgt 1 : 1, HOSu oder HOBt wird als ein Aktivierungsmittel eingesetzt; ein Verhältnis der Menge des Aktivierungsmittels zu dem Fragment 1 beträgt 1 : 1; und DCM, THF, DMF oder DMSO wird als Lösemittel eingesetzt.

5. Verfahren zur Herstellung von Oxytocin durch Flüssigphasen-Polypeptidsynthese gemäß Anspruch 1, wobei die Verbindung 5 in Schritt (5) Boc-Cys(Acm)-Tyr(tBu)-Ile-Gln(Trt)-Asn(Trt)-Cys(Acm)-Pro-OH ist und die Synthese der Verbindung 5 die folgenden Schritte umfasst:
LiOH oder NaOH wird als Verseifungsreagenz eingesetzt, dessen Menge 5-15 Äquivalente der Verbindung 4 beträgt; eine Konzentration ist 0,05 - 0,2 M; und THF, DMF, Methanol, Acetonitril oder DMSO wird als Lösemittel eingesetzt.

6. Verfahren zur Herstellung von Oxytocin durch Flüssigphasen-Polypeptidsynthese gemäß Anspruch 1, wobei die Verbindung 6 in Schritt (6) Boc-Cys(Acm)-Tyr(tBu)-Ile-Gln(Trt)-Asn(Trt)-Cys(Acm)-Pro-Leu-Gly-NH₂ ist; und die Synthese der Verbindung 6 die folgenden Schritte umfasst:
ein Verhältnis der Menge der Verbindung 5 zu der Menge des Fragmentes 3 beträgt 1 : 1 : 1,05 - 2; DCC, DIC, BOP oder ein Azidreagenz wird als Kondensationsmittel eingesetzt; HOSu, HOOBt oder HOBt wird als Aktivierungsmittel eingesetzt; und THF, DMF oder DMSO wird als Lösemittel eingesetzt.

7. Verfahren zur Herstellung von Oxytocin durch Flüssigphasen-Polypeptidsynthese gemäß Anspruch 1, wobei in Schritt (7) die Verbindung 7 unter Verwendung von Jod als Reagenz synthetisiert wird, eine Menge des Jods 5-10 Äquivalente der Verbindung 6 beträgt und eine Konzentration des Jods 0,1 - 0,5 M ist, und DMF wird als Lösemittel eingesetzt wird; nach Beendigung der Reaktion 0,1-%-ige Natriumthiosulfatlösung verwendet wird, um den Feststoff abzutrennen, dann mit Wasser gewaschenund vakuumgetrocknet wird, um die Verbindung 7 zu erhalten.

## Revendications

1. Procédé de fabrication de l'oxytocine par la synthèse de polypeptide en phase liquide, qui comprend les étapes suivantes de:
(1) synthétiser un fragment 1 : Boc-Cys(Acm)-Tyr(tBu)-OH;
(2) synthétiser un fragment 2 : H-Ile-Gln(Trt)-Asn(Trt)-Cys(Acm)-Pro-OMe:
(3) synthétiser un fragment 3 : H-Leu-Gly-NH₂;
(4) synthétiser un composé 4 : Boc-Cys(Acm)-Tyr(tBu)-Ile-Gln(Trt)-Asn(Trt)-Cys(Acm)-Pro-OMe;
(5) synthétiser un composé 5 : Boc-Cys(Acm)-Tyr(tBu)-Ile-Gln(Trt)-Asn(Trt)-Cys(Acm)-Pro-OH;
(6) synthétiser un composé 6 : Boc-Cys(Acm)-Tyr(tBu)-Ile-Gln(Trt)-Asn(Trt)-Cys(Acm)-Pro-Leu-Gly-NH₂;
(7) synthétiser un composé 7 : 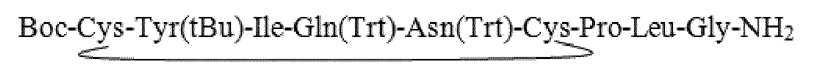
Boc-Cys-Tyr(tBu)-Ile-Gln(Trt)-Asn(Trt)-Cys-Pro-Leu-Gly-NH2

 en utilisant du iode comme réactif,
(8) déprotéger le composé 7 avec de l'acide trifluoroacétique et concentrer le mélange de réaction pour obtenir une solution d'oxytocine brute ;
(9) purifier le produit brut par moyen de la chromatographie de gel de silice en phase inverse C18 pour obtenir de l'oxytocine pure ;
dans lequel la synthétisation du fragment 2 comprend les étapes suivantes de :
(1) synthétiser le composé 2.1 : Fmoc-Cys(Acm)-Pro-OMe;
un rapport entre une quantité de Fmoc-Cys(Acm)-OH et une quantité de H-Pro-OMe.HCl est de 1 : 1,05 à 1 : 1,5 ; DIC ou DCC est utilisé comme un agent de condensation ; un rapport entre une quantité de l'agent de condensation et la quantité de H-Pro-OMe.HCl est 1 : 1 ; HOSu ou HOBt ou HOOBt est employé comme un activateur ; un rapport entre une quantité de l'activateur et la quantité de H-Pro-OMe.HCl est 1 : 1 ; le triéthylamine (TEA), le diisopropyléthylamine (DIPEA) ou le N-méthylmorpholine (NMM) est employé comme une base ; un rapport entre une quantité de la base et la quantité de H-Pro-OMe.HCl est 1 : 1 ; et DMF, DMSO, DCM ou THF est utilisé comme un solvant ;
(2) synthétiser le composé 2.2 : H-Cys(Acm)-Pro-OMe :
déprotéger le composé 2.1 avec le diéthylamine (DEA) ou le 5% pipérazine/DCM comme un agent de déprotection, concentrer après l'achèvement de la réaction de déprotection, utiliser de l'éther de pétrole pour séparer le produit, filtrer et sécher sous vide pour obtenir le composé 2.2 ;
(3) synthétiser le composé 2.3 : Fmoc-Asn(Trt)-Cys(Acm)-Pro-OMe :
un rapport entre une quantité du composé 2.2 et une quantité de Fmoc-Asn(Trt)-OH est de 1 : 1,05 à 1 : 1,5 ; DIC ou DCC est utilisé comme un agent de condensation, un rapport entre une quantité de l'agent de condensation et la quantité de Fmoc-Asn(Trt)-OH est 1 : 1 ; HOSu, HOOBt ou HOBt est employé comme un activateur ; un rapport entre une quantité de l'activateur et la quantité de Fmoc-Asn(Trt)-OH est 1 : 1 ; et DMF, DMSO, DCM ou THF est utilisé comme un solvant ;
(4) synthétiser le composé 2.4 : H-Asn(Trt)-Cys(Acm)-Pro-OMe :
déprotéger le composé 2.3 avec le diéthylamine (DEA) ou le 5% pipérazine/DCM comme un agent de déprotection, concentrer après l'achèvement de la réaction de déprotection, utiliser une solution de NaHCO₃ saturée pour séparer le produit, filtrer, laver avec de l'eau jusqu'à la neutralisation et sécher sous vide, et ensuite laver le produit brut avec l'éther de pétrole en agitant, en filtrant et en séchant sous vide pour obtenir le composé 2.4,
(5) synthétiser le composé 2.5 : Fmoc-Gln(Trt)-Asn(Trt)-Cys(Acm)-Pro-OMe :
un rapport entre une quantité du composé 2.4 et une quantité de Fmoc-Gln(Trt)-OH est de 1 : 1,05 à 1 : 1,5 ; DIC ou DCC est utilisé comme un agent de condensation ; un rapport entre une quantité de l'agent de condensation et la quantité de Fmoc-Gln(Trt)-OH est 1 : 1 ; HOSu, HOOBt ou HOBt est employé comme un activateur ; un rapport entre une quantité de l'activateur et la quantité de Fmoc-Gln(Trt)-OH est 1 : 1 ; et DMF, DMSO, DCM ou THF est utilisé comme un solvant ;
(6) synthétiser le composé 2.6 : H-Gln(Trt)-ASn(Trt)-Cys(Acm)-Pro-OMe :
déprotéger le composé 2.5 avec le diéthylamine (DEA) ou le 5% pipérazine/DCM comme un agent de déprotection, concentrer après l'achèvement de la réaction de déprotection, utiliser une solution de NaHCO₃ saturée pour séparer le produit, filtrer, laver avec de l'eau jusqu'à la neutralisation et sécher sous vide, et ensuite laver le produit brut avec l'éther de pétrole en agitant, en filtrant et en séchant sous vide pour obtenir le composé 2.6 ;
(7) synthétiser le composé 2.7 : Fmoc-Ile-Gln(Trt)-Asn(Trt)-Cys(Acm)-Pro-OMe :
un rapport entre une quantité du composé 2.6 et une quantité de Fmoc-Ile-OH est de 1 : 1,05 à 1 : 1,5 ; DIC ou DCC est utilisé comme un agent de condensation ; un rapport entre une quantité de l'agent de condensation et la quantité de Fmoc-Ile-OH est 1 : 1 ; HOSu, HOOBt ou HOBt est employé comme un activateur ; un rapport entre une quantité de l'activateur et la quantité de Fmoc-Ile-OH est 1 : 1 ; et DMF, DMSO, DCM ou THF est utilisé comme un solvant ;
(8) synthétiser le fragment 2 : H-Ile-Gln(Trt)-Asn(Trt)-Cys(Acm)-Pro-OMe :
déprotéger le composé 2.7 avec le diéthylamine (DEA) ou le 5% pipérazine/DCM comme un agent de déprotection, concentrer après l'achèvement de la réaction de déprotection, utiliser une solution de NaHCO₃ saturée pour séparer le produit, filtrer, laver avec de l'eau jusqu'à la neutralisation et sécher sous vide, et ensuite laver le produit brut avec l'éther de pétrole en agitant, en filtrant et en séchant sous vide pour obtenir le fragment 2.

2. Procédé de fabrication de l'oxytocine par la synthèse de polypeptide en phase liquide selon la revendication 1, dans lequel le fragment 1 dans l'étape (1) est : Boc-Cys(Acm)-Tyr(tBu)-OH ; les unités d'acide aminé pour synthétiser le fragment 1 sont Boc-Cys-(Acm)-OH et H-Tyr(tBu)-OH, DIC ou DCC est utilisé comme un agent de condensation ;HOSu ou HOBt est utilisé comme un activateur ; THF et de l'eau sont utilisés comme un solvant ; et le rapport entre THF et l'eau est de 1 :1 à 5 : 1, le solvant pouvant aussi être DMF ou DMSO.

3. Procédé de fabrication de l'oxytocine par la synthèse de polypeptide en phase liquide selon la revendication 1, dans lequel le fragment 3 dans l'étape (3) est : H-Leu-Gly-NH₂ ; et le fragment 3 peut être synthétisé par la solution 1 ou la solution 2 comme suivant :
solution 1 : Boc-Leu-OH et H-Gly-NH₂.HCl sont utilisés comme des unités d'acide aminé ; BOP, HBTU, TBTU, DIC ou DCC/HOBt ou HOSu est utilisé comme un agent de condensation ; TEA, NMM ou DIPEA est utilisé comme une base ; DMF, DMSO, THF ou DCM est utilisé comme un solvant ; synthétiser Boc-Leu-Gly-NH₂ et ensuite enlever le groupe de protection boc de celui-ci avec du TFA pour obtenir H-Leu-Gly-NH₂ ;
solution 2 : Fmoc-Leu-OH, H-Gly-NH₂.HCl sont utilisés comme des unités d'acide aminé ; BOP, HBTU, TBTU, DIC ou DCC/HOBt ou HOSu est utilisé comme un agent de condensation ; TEA, NMM ou DIPEA est utilisé comme une base ; DMF, DMSO, THF ou DCM est utilisé comme un solvant ; après avoir synthétisé Fmoc-Leu-Gly-NH₂ enlever le groupe de protection Fmoc de celui-ci pour obtenir H-Leu-Gly-NH₂ ; et un agent de déprotection est le diéthylamine (DEA) ou le 5% pipérazine/DCM.

4. Procédé de fabrication de l'oxytocine par la synthèse de polypeptide en phase liquide selon la revendication 1, dans lequel le composé 4 dans l'étape (4) est : Boc-Cys(Acm)-Tyr(tBu)-lle-Gln(Trt)-Asn(Trt)-Cys(Acm)-Pro-OMe et la synthèse du composé 4 comprend les étapes suivantes :
un rapport entre une quantité du fragment 1 et une quantité du fragment 2 est 1,05-1,5 : 1; BOP, DCC, DIC ou un réactif d'azide est utilisé comme un agent de condensation ; un rapport entre une quantité de l'agent de condensation et la quantité du fragment 1 est 1 : 1 ; HOSu ou HOBt est employé comme un activateur ; un rapport entre une quantité de l'activateur et la quantité du fragment 1 est 1 : 1 ; et DCM, THF, DMF ou DMSO est utilisé comme un solvant.

5. Procédé de fabrication de l'oxytocine par la synthèse de polypeptide en phase liquide selon la revendication 1, dans lequel le composé 5 dans l'étape (5) est : Boc-Cys(Acm)-Tyr(tBu)-lle-Gln(Trt)-Asn(Trt)-Cys(Acm)-Pro-OH et la synthèse du composé 5 comprend les étapes suivantes :
LiOH ou NaOH est utilisé comme un réactif de saponification, dont une quantité est 5-15 équivalents du composé 4 ; une concentration est 0,05-0,2 M ; et THF, DMF, du méthanol, de l'acétonitrile ou DMSO est utilisé comme un solvant.

6. Procédé de fabrication de l'oxytocine par la synthèse de polypeptide en phase liquide selon la revendication 1, dans lequel le composé 6 dans l'étape (6) est : Boc-Cys(Acm)-Tyr(tBu)-Ile-Gln(Trt)-Asn(Trt)-Cys(Acm)-Pro-Leu-Gly-NH₂ ; et la synthèse du composé 6 comprend les étapes suivantes :
un rapport entre une quantité du composé 5 et une quantité du fragment 3 est 1 : 1 : 1,05-2; DCC, DIC, BOP ou un réactif d'azide est utilisé comme un agent de condensation ; HOSu, HOOBt ou HOBt est employé comme un activateur ; et THF, DMF ou DMSO est utilisé comme un solvant.

7. Procédé de fabrication de l'oxytocine par la synthèse de polypeptide en phase liquide selon la revendication 1, dans lequel dans l'étape (7) le composé 7 est synthétisé en utilisant de l'iode comme un réactif, une quantité de l'iode est 5-10 équivalents du composé 6 et une concentration de l'iode est 0,1-0,5 M ; et DMF est utilisé comme un solvant, après l'achèvement de la réaction on utilise 0,1 % solution de thiosulfate de sodium pour séparer le solide, on lave avec de l'eau et sèche sous vide pour obtenir le composé 7.
